# EUROPEAN PATENT APPLICATION

(11) **EP 2 072 005 A1**
(43) Date of publication of application: **24.06.2009**
(21) Application number: 07730474.9
(22) Date of filing: 26.04.2007
(51) Int. Cl.: A61B 5/00

(54) **SYSTEM FOR DETERMINING AND MONITORING DESATURATION INDICES AND INSTANTANEOUS RESPIRATORY RATE**

(30) Priority: 10.10.2006 ES 200602577
(71) Applicant: Universidad De Cádiz, 11001 Cádiz (ES)
(72) Inventor: ROJAS OJEDA, Juan Luis, 11002 Cádiz (ES); LEÓN JIMÉNEZ, Antonio, 11003 Cádiz (ES); CRESPO FOIX, Luis Felipe, 11002 Cádiz (ES); GROSS, Nicole, 11002 Cádiz (ES); SANCHEZ MORILLO, Daniel, 11002 Cádiz (ES)
(74) Representative: ABG Patentes, S.L.
(86) International application number: PCT/ES2007/000253
(87) International publication number: WO 2008/043864

(57) **Abstract**

The invention relates to a system for determining and monitoring desaturation indices and instantaneous respiratory rate, based on extracting components from the blood oxygen saturation (SpO₂) signal captured by an oximeter, obtaining and processing the data in the frequency domain in order to detect respiratory events and determine values such as respiratory rate and deviations therefrom (tachypnea/bradypnea) and desaturation indices.

It is therefore an invention linked to the field of bioengineering, with applications in the field of medicine, allowing monitoring and assisting with the diagnosis of respiratory disorders for its use in anesthesia, intensive care units and healthcare emergencies and assisting in the diagnosis of sleep apnea/hypopnea syndrome (SAHS).

## Description

### Technical Field of the Invention

The system object of this patent is based on extracting components by means of the frequency analysis of the signal captured exclusively by an oximeter, obtaining and processing information from blood oxygen saturation (SpO₂) data. It is therefore an invention linked to the field of bioengineering, with applications in the field of medicine, allowing monitoring and assisting with the diagnosis of respiratory disorders, for its use in hospitals and in the home, and for assisting with the diagnosis of sleep apnea-hypopnea syndrome (SAHS).

### State of the Art and Background of the Invention

Oximetry is essentially based on the so-called Beer-Lambert law, which allows calculating the concentration of a substance in solution from its optical absorption at a certain wavelength. In the case of blood, there are two substances relevant to oxygenation, which are hemoglobin (Hb) and oxyhemoglobin (HbO₂). Since the deoxygenation of blood causes an increasing absorption in the red band and a decreasing absorption in the infrared band, oximeters therefore have two wavelengths: red and infrared wavelengths, which allow distinguishing oxygenated hemoglobin from reduced hemoglobin. There are different types of equipment on the market the operation principle of which is always the same.

The approach to the diagnosis of sleep apnea-hypopnea syndrome (SAHS) by means of determining the oxygen desaturation event index, comparing the oxygen saturation (SpO₂) values with the baseline of the patient or with a theoretical baseline corresponding to healthy patients, is frequent.

The rigorous interpretation of nocturnal oximetry requires knowing the normal oxygen saturation values during sleep.

Based on these clinical criteria, the works aimed at automated diagnosis establish several criteria for detecting events and calculating desaturation indices. Those based on measuring SpO₂ establish, to the best of the inventors' knowledge, two types of essential criteria which in turn apply different calculation methods, obtaining different results. In the cases of severe SAHS, they do not differ substantially, but in mild and moderate cases they can lead to possible errors in the diagnosis. In relation to the algorithms used by the manufacturers of measurement instruments for determining desaturation indices, to the best of the inventors' knowledge, they are not provided to the user, therefore it is difficult to establish reliable comparative criteria.

Obtaining the DI (desaturation index) requires establishing a reference level with respect to which the events occurring are determined. Obtaining this reference level or baseline level has given rise to several methods which are considered in this study as a reference for obtaining events.

There are basically two strategies which have been established:
- Methods based on the deviation with respect to a certain baseline level.
- Methods based on the decrease or increase speed (flanks).

The first group includes those algorithms in which the baseline level is obtained as a statistical average of the SpO₂ values during the total recorded during the experiment [4]. The baseline level is obtained as an average of the signal during the complete period of the experiment, excluding artifacts and faults in the measurement. For the detection of respiratory events, this approach is based on searching for decreases below 4% of this baseline level. In other algorithms, the baseline level is a moving average of the values located in the 95^{th} percentile for one or several minutes before the treated instant. Therefore, before the calculation of the moving average, all the data of the treated period will have been subjected to a statistical filtering eliminating those values less than that corresponding to the 95^{th} percentile. This ensures high baseline level values leading to good practical results in the discrimination of respiratory events [3,4]. As a result of the applications of the percentile approach to filtering, the desaturation periods in the patient are automatically excluded in the calculation of the baseline level, and the dynamics of the time evolution of the oximetry of the patient are furthermore conserved [2].

The second group takes into account that the apnea events can be completely located below the standard baseline level of 90% and that even when the baseline level is a statistical average for each patient, a considerable number of desaturations cannot be detected due to the fact that the SpO₂ recoveries do not reach the threshold marked by the baseline. This situation leads to error in the calculation of the DI.

Rauscher [4] proposes 2 new methods which do not require determining the baseline level, since it only considers level increases or decreases during a certain time interval, or in other words, it exclusively considers the average slope of the SpO₂ signal during defined time intervals. According to this approach, Rauscher obtains event rates by means of detecting the number of decreases greater than 4% during a time interval not less than 40 seconds, and by means of detecting the number of recoveries greater than 3% in a time interval of at least 10 seconds. This process justifies the difference in the up- and down-slopes, adducing that the restorations respond more quickly to the restoration of the respiratory activity.

However, these statistical assessment measurements of the baseline or baseline level of blood oxygen saturation can be affected by the following elements altering the measurement based on the time domain [1]:
- Low blood circulation level
- High HbCO fraction (e.g.: smokers or people in toxic environments)
- Deoxyhemoglobin
- Anemic hypoxemia

To the best of the inventors' knowledge, respiratory rates (bradypneas, tachypneas and normal respiration) are calculated through oronasal flow cannulas, extensometric gauges, accelerometers and other indirect measurement devices, and neither patents nor literature for measurement based on exclusively using oximetry have been found.

### Literature

[1] Technik in der Kardiologie. A.Bolz, W.Urbaszek.Springer, 2001.
[2] Eusebi Chiner, Jaime Signes-Costa, Juan Manuel Arriero, et al. Nocturnal oximetry for the diagnosis of the sleep apnoea hypopnoea syndrome: a method to reduce the number of polysomnographies? THORAX 1999;54:968-
[3] J. C. Vázquez, W. H. Tsai, W. W. Flemons, A. Masuda, R. Brant, E. Hajduk, W.A. Whitelaw, J. E. Remmers. Automated analysis of digital oximetry in the diagnosis of obstructive sleep apnea. THORAX, vol. 55, pp. 302-307, 2000.
[4] Rauscher, H., Popp, W., and Zwick, H. Computerized detection of respiratory events during sleep from rapid increases in oxyhemoglobin saturation. LUNG 1991, 169:335-342.

### Description of the Invention

The method object of this patent is based on extracting frequency components from the signal captured by an oximeter, obtaining information from vital respiratory physiological data, for assisting with the automated diagnosis of problems related to hypoxia in general and the sleep apnea-hypopnea syndrome in particular, independently of the baseline and absolute SpO₂ values.

The invention allows:
- Integration of the calculation of the DI (desaturation index) and of the respiratory rhythms of the patient (normal, bradypneas and tachypneas) in current oximetry equipment in a novel manner, independently of the baseline and absolute values of the patient.
- Processing of the captured data, by means of a microprocessor-based system, to extract and display the mentioned physiological variables: Spectro-oximetry and Spectro-apneas.
- Display of parameters resulting from the analysis of the previous variables: respiratory rhythm and desaturation index/hour.
- The application at home by storing or transmitting the data for its interpretation by a specialist.

### Brief Description of the Drawings

To make the object of the invention more intelligible, it has been illustrated with three schematic figures serving as a demonstrative example:

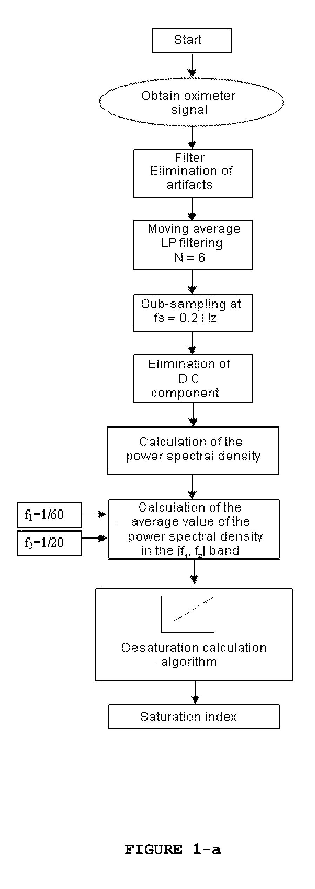

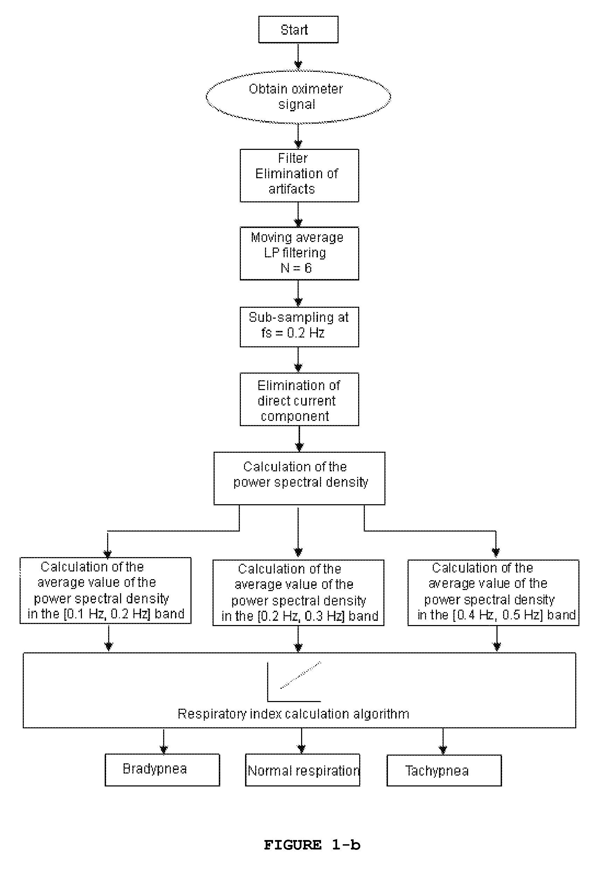

Figure 1.a shows the different information processing steps until the generation of the information about the desaturation index.
Figure 1.b shows the different information processing steps until the generation of the information about the respiratory indices.

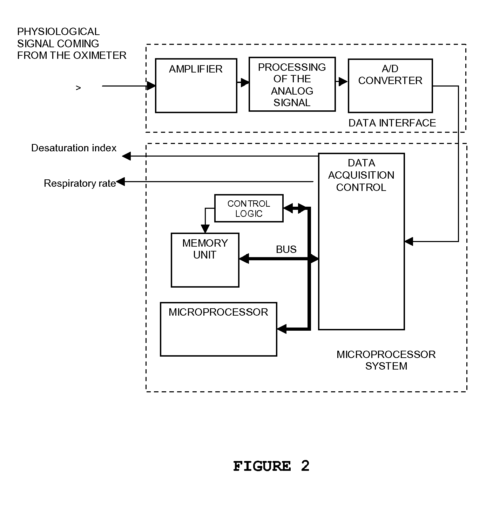

Figure 2 schematically depicts the physical system on which the invention is based.
   Accompanying these 2 figures, there are another four figures attached which graphically show the results expressed herein and which allow reliably comparing the information expressed in the description of the invention below.

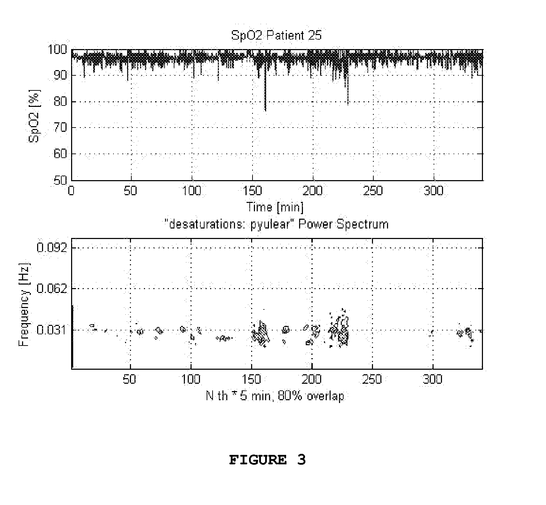

Figure 3 shows the form of the data captured by the oximeter and the corresponding spectral power of the oxygen desaturations (SpO₂) in the case of a patient with a diagnosis of medium desaturations (DI= 25).

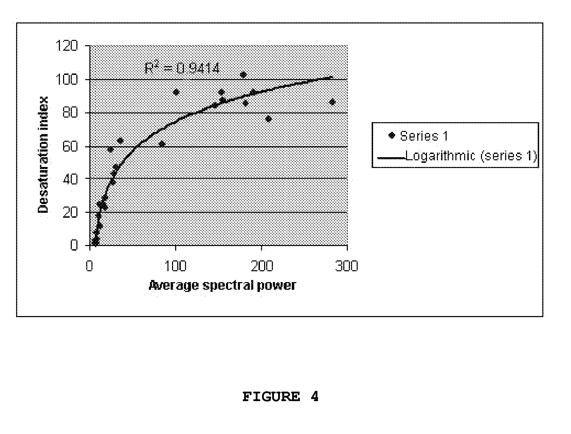

Figure 4 shows the correlation existing between the calculated average spectral power in the ranges established for oxygen desaturations, and the corresponding desaturation indices established by the experts of the reference hospital.

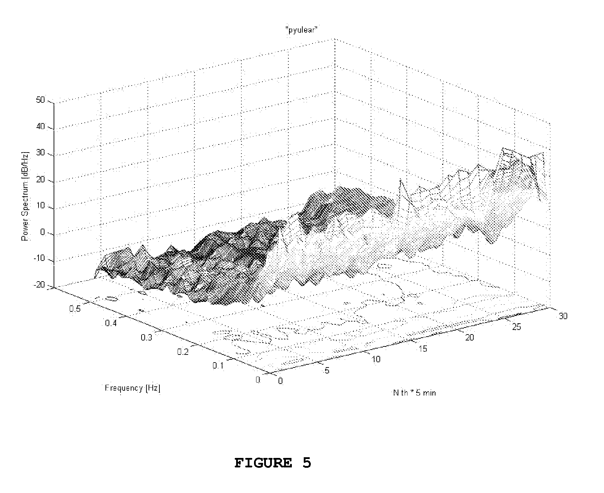

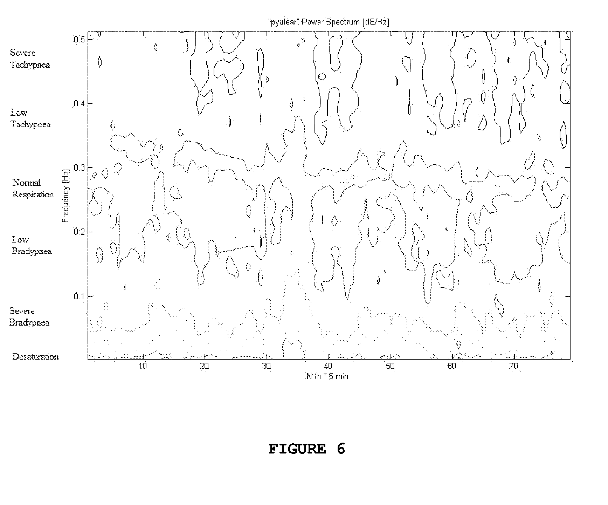

Figures 5 and 6 show the spectral location in the frequency ranges of the respiratory components and of the desaturations.

### Detailed Description of the Invention

In relation to the invention described, it has two differentiated parts; a method is provided for calculating the desaturation index based on frequency analysis, therefore the method is not affected by the calculation of baselines used in traditional methods, or by the existence of artifacts outside or inside the patient which can modify the measurement. Likewise, a method which is not included in current oximeters is described for determining the respiratory rhythms of patients, also based on the frequency analysis of the respiratory rhythms detected in the signals provided by the oximeter.

The invention described and set forth consequently involves a simplification of the tests for the diagnosis of certain dysfunctions associated with respiratory disorders such as the obstructive sleep apnea-hypopnea syndrome (SAHS), providing an aid for the diagnosis of respiratory disorders and for evaluation in risk situations.

The following are emphasized among the advantages provided with respect to the current state of the art:
1. System with a simple application and operation.
2. It does require calibrating the measurement.
3. It does not require skilled personnel
4. Use at home and use in hospital
5. Use in disaster and emergency situations for the quick discrimination of the vital situation of the affected people
6. Novel processing of the captured information for information useful for the diagnosis.

The oximetry sensor provides an electrical signal, proportional to arterial oxyhemoglobin saturation (SaO₂). This electric signal is transmitted to a processing circuitry, which amplifies the signal, filters it and converts it into a digital signal. The filtering parameters prior to the A/D conversion are conditioned by the sampling frequency. This filtering can be implemented through hardware or software or by means of a combination of both. The resulting digital signal is delivered to a microprocessor system for its evaluation.

This system will operate according to instructions stored in memory, implementing the calculation process shown in Figures 1-a and 1-b. Furthermore, the system may store the data captured and obtained through the processing in a memory. The storage can be carried out in any storage system or combination thereof, such as volatile memories (DRAM), non-volatile memories, hard drives, CD-RW, DVD, removable memories (SD, MMC cards,...). The microprocessor system can furthermore display the results to the operator through a display, generate acoustic alerts, luminous alerts or alerts of any other type. It can contain input devices such as touch screens, keyboards, or any other device intended for the input of information by the operator.

It is evident that this microprocessor system can be physically implemented by one or several devices, capable of fulfilling the described functions. They can be general or specific purpose systems such as microprocessors, microcontrollers, digital signal processors, application-specific integrated circuits (ASICs), personal computers, PDAs, smartphones, ...

It is important to emphasize that the processing for obtaining the respiratory and desaturation indices is physically decoupled and can be made independent. In contrast, the preprocessing steps for the signal captured by the oximeter are identical. The collected signal is subjected to an initial filtering to eliminate artifacts in the measurement. A low-pass filtering is subsequently applied by a moving average filter, with a sample index for the average which can vary about 5 samples. The output of this filter is subjected to a sub-sampling to generate a cluster of samples at fs=0.2 Hz, from which the DC component is eliminated. The pre-processing block thus ends.

Both for obtaining the respiratory indices and for calculating the desaturation index, the power spectral density of the signal resulting from the previous preprocessing is calculated, using to that end any of the methods described in the literature (parametric or non-parametric).

For the calculation of the desaturation index, the average value of the previous spectral estimation in the [1/60 Hz, 1/20 Hz] band is calculated. This average value allows directly obtaining the DI value through the logarithmic ratio statistically linking both amounts, according to the adjustment performed with a control group. The desaturation index is stored by the system.

For the calculation of the respiratory indices, the average value of the spectral estimation in the [0.1 Hz, 0.2 Hz], [0.2 Hz, 0.3 Hz], [0.4 Hz, 0.5 Hz] bands is calculated. These average values again allow directly obtaining the normal respiration, bradypnea and tachypnea index values through the ratio statistically linking the respective amounts, according to the adjustment performed with a control group. The normal respiration, bradypnea and tachypnea indices are stored by the system.

### Embodiment of the Invention

The method includes the following phases:
1. Test for collecting data from the patient, with the placement of the oximetry sensor.
2. The acquisition system conditions the signal by means of a preamplifier amplifier and anti-aliasing filter. The sampling is done with frequencies not less than 1 Hz. The obtained data is stored in a record for its processing.
3. A prior filtering of the previous work space is applied to eliminate artifacts in the measurement, generating a new fault-free data record. This preprocessing can include the truncation or the interpolation on the original record.
4. A moving average (LP) filtering is applied, followed by a sub-sampling at a new rate of 0.2 Hz. The DC component is eliminated from the resulting signal.
5. Processing of the signal, to extract the desaturation index. The power spectral density is calculated and its average value in the [1/60, 1/20 Hz] band is evaluated.
6. Delivery of the result of the processing to a decision-making step, previously adjusted with a control group, to directly obtain the desaturation index from the previous average spectral value.
7. The desaturation index is determined immediately and can be presented to the patient by his or her specialist doctor in real time or as soon as the test ends.
8. Processing of the signal, to extract the respiratory indices. The power spectral density is calculated and its average value in the [0.1, 0.2 Hz], [0.2, 0.3 Hz] and [0.4, 0.5 Hz] bands is evaluated.
9. Delivery of the result of the processing to a decision-making step, previously adjusted with a control group, to directly obtain the bradypnea index, the normal respiration index and the tachypnea index from the previous average spectral values.
10. The bradypnea index, the normal respiration index and the tachypnea index are determined immediately and can be presented to the patient by his or her specialist doctor in real time or as soon as the test ends.

## Claims

1. A system for determining and monitoring desaturation indices and instantaneous respiratory rate, **characterized in that** it is performed by means of the frequency analysis of the signal captured by an oximeter, obtaining and processing information from blood oxygen saturation data.

2. The system for determining and monitoring desaturation indices and instantaneous respiratory rate according to claim 1, **characterized in that** it integrates, together with the current processes for recording blood oxygen saturation (SpO₂) data, a system with processing and analysis capacity with a method for obtaining the desaturation index and the respiratory rate, without needing to calibrate the data.

3. The system for determining and monitoring desaturation indices and instantaneous respiratory rate according to claims 1 and 2, **characterized in that** the general method used comprises the following phases:
a. Test for collecting data from the patient.
b. Filtering of the obtained data to eliminate artifacts in the measurement, standardizing and generating a new fault-free data record.
c. Processing of the signal, to obtain the power spectral density in order to extract the variables related to the desaturation and the respiratory rate indices, contained in the captured signal.
d. Delivery of the result of the processing to a decision-making step to generate output information, with useful guidelines for the specialist to facilitate the diagnosis.

4. The system for determining and monitoring desaturation indices and instantaneous respiratory rate according to claims 1 to 3, **characterized in that** the specific method for calculating the desaturation index used comprises the following phases:
a. Test for collecting data from the patient by means of the oximetry sensor.
b. Conditioning of the signal by means of a preamplifier amplifier and anti-aliasing filter, performing the sampling with frequencies not less than 0.2 Hz and storing the data obtained in a record for its processing.
c. Application of a prior filtering to eliminate artifacts in the measurement, generating a new fault-free data record, which can include the truncation or the interpolation on the original record.
d. Moving average (LP) filtering, followed by a sub-sampling at a new rate, eliminating the DC component from the resulting signal.
e. Processing of the signal to extract the desaturation index, the power spectral density being calculated and its average value in the [1/60, 1/20 Hz] band being evaluated.
f. Delivery of the result of the processing to a decision-making step, previously adjusted with a control group, to directly obtain the desaturation index from the previous average spectral value.
g. Immediate determination of the desaturation index, which can be presented to the patient by his or her specialist doctor in real time or as soon as the test ends.

5. The system for determining and monitoring desaturation indices and instantaneous respiratory rate according to claims 1 to 3, **characterized in that** the specific method for calculating the respiratory indices used comprises the following phases:
a. Test for collecting data from the patient by means of the oximetry sensor.
b. Conditioning of the signal by means of a preamplifier amplifier and anti-aliasing filter, performing the sampling with frequencies not less than 0.2 Hz and storing the data obtained in a record for its processing.
c. Application of a prior filtering to eliminate artifacts in the measurement, generating a new fault-free data record, which can include the truncation or the interpolation on the original record.
d. Moving average (LP) filtering, followed by a sub-sampling at a new rate, eliminating the DC component from the resulting signal.
e. Processing of the signal to extract the respiratory indices, the power spectral density being calculated and its average value in the [0.1, 0.2 Hz], [0.2, 0.3 Hz] and [0.4, 0.5 Hz] bands being evaluated.
f. Delivery of the results of the processing to a decision-making step, previously adjusted with a control group, to directly obtain the bradypnea index, the normal respiration index and the tachypnea index from the previous average spectral values.
g. Immediate determination of the bradypnea index, the normal respiration index and the tachypnea index, which can be presented to the patient by his or her specialist doctor in real time or as soon as the test ends.

6. A use of the system for determining and monitoring desaturation indices and instantaneous respiratory rate described in claims 1 to 5, for assisting with the diagnosis of sleep apnea-hypopnea syndrome and of other respiratory disorders.

7. A use of the system for determining and monitoring desaturation indices and instantaneous respiratory rate described in claims 1 to 5, for the use in disaster and emergency situations for the quick discrimination of the vital situation of the affected people based on the study of their respiratory rates.

## Amended claims

### Amended claims under Art. 19.1 PCT

**1.** A method for determining and monitoring physiological parameters by means of the frequency analysis of the signal captured by an oximeter, **characterized in that** the calculation of the desaturation index used comprises the following phases:
a. Test for collecting data from the patient by means of the oximetry sensor.
b. Conditioning of the signal by means of a preamplifier amplifier and anti-aliasing filter, performing the sampling with frequencies not less than 0.2 Hz and storing the data obtained in a record for its processing.
c. Application of a prior filtering to eliminate artifacts in the measurement, generating a new fault-free data record, which can include the truncation or the interpolation on the original record.
d. Moving average (LP) filtering, followed by a sub-sampling at a new rate, eliminating the DC component from the resulting signal.
e. Processing of the signal to extract the desaturation index, the power spectral density being calculated and its average value in the [1/60, 1/20 Hz] band being evaluated.
f. Delivery of the result of the processing to a decision-making step, previously adjusted with a control group, to directly obtain the desaturation index from the previous average spectral value.
g. Immediate determination of the desaturation index, which can be presented to the patient by his or her specialist doctor in real time or as soon as the test ends.

**2.** A method for determining and monitoring for determining and monitoring physiological parameters by means of the frequency analysis of the signal captured by an oximeter, **characterized in that** the calculation of the respiratory indices used comprises the following phases:
a. Test for collecting data from the patient by means of the oximetry sensor.
b. Conditioning of the signal by means of a preamplifier amplifier and anti-aliasing filter, performing the sampling with frequencies not less than 0.2 Hz and storing the data obtained in a record for its processing.
c. Application of a prior filtering to eliminate artifacts in the measurement, generating a new fault-free data record, which can include the truncation or the interpolation on the original record.
d. Moving average (LP) filtering, followed by a sub-sampling at a new rate, eliminating the DC component from the resulting signal.
e. Processing of the signal to extract the respiratory indices, the power spectral density being calculated and its average value in the [0.1, 0.2 Hz], [0.2, 0.3 Hz] and [0.4, 0.5 Hz] bands being evaluated.
f. Delivery of the results of the processing to a decision-making step, previously adjusted with a control group, to directly obtain the bradypnea index, the normal respiration index and the tachypnea index from the previous average spectral values.
g. Immediate determination of the bradypnea index, the normal respiration index and the tachypnea index, which can be presented to the patient by his or her specialist doctor in real time or as soon as the test ends.

**3.** A use of the method for determining and monitoring physiological parameters by means of the frequency analysis of the signal captured by an oximeter described in claims 1 and 2, for assisting with the diagnosis of sleep apnea-hypopnea syndrome and of other respiratory disorders.

**4.** A use of the method for determining and monitoring physiological parameters by means of the frequency analysis of the signal captured by an oximeter described in claims 1 and 2, for the use in disaster and emergency situations for the quick discrimination of the vital situation of the affected people based on the study of their respiratory rates.
